# EUROPÄISCHE PATENTSCHRIFT

(11) **EP 1 109 619 B1**
(45) Veröffentlichungstag und Bekanntmachung des Hinweises auf die Patenterteilung: **18.12.2002**
(21) Anmeldenummer: 99941531.8
(22) Anmeldetag: 06.08.1999
(51) Int. Cl.: B01J 8/06, C07C 51/25

(54) **REAKTOR MIT EINEM KONTAKTROHRBÜNDEL**
REACTOR COMPRISING A CONTACT TUBE BUNDLE
REACTEUR COMPORTANT UN FAISCEAU DE TUBES DE CONTACT

(30) Priorität: 13.08.1998 DE 19836792
(43) Veröffentlichungstag der Anmeldung: 27.06.2001
(73) Patentinhaber: BASF AKTIENGESELLSCHAFT, 67056 Ludwigshafen (DE)
(72) Erfinder: CORR, Franz, D-67067 Ludwigshafen (DE); OLBERT, Gerhard, D-69221 Dossenheim (DE)
(74) Vertreter: Isenbruck, Günter, Dr.
(86) Internationale Anmeldenummer: EP9905701
(87) Internationale Veröffentlichungsnummer: WO00009253

(56) Entgegenhaltungen:
- GB-A- 553 107
- US-A- 2 098 148
- US-A- 3 760 870
- US-A- 3 871 445
- US-A- 5 228 315

## Beschreibung

Die Erfindung betrifft einen Reaktor mit einem Kontaktrohrbündel, durch dessen die Kontaktrohre umgebenden Raum ein Wärmetauschmittelkreislauf geleitet wird, sowie die Verwendung des Reaktors zur Durchführung von Oxidationsreaktionen.

Die übliche Bauart gattungsgemäßer Reaktoren besteht aus einem, in der Regel zylinderförmigen Behälter, in dem ein Bündel, d.h. eine Vielzahl von Kontaktrohren in üblicherweise vertikaler Anordnung untergebracht ist. Diese Kontaktrohre, die gegebenenfalls geträgerte Katalysatoren enthalten können, sind mit ihren Enden in Rohrböden abdichtend befestigt und münden in jeweils eine am oberen bzw am unteren Ende mit dem Behälter verbundene Haube. Über diese Hauben wird das die Kontaktrohre durchströmende Reaktionsgemisch zu- bzw. abgeführt. Durch den die Kontaktrohre umgebenden Raum wird ein Wärmetauschmittelkreislauf geleitet, um die Wärmebilanz, insbesondere bei Reaktionen mit starker Wärmetönung, auszugleichen.

Aus wirtschaftlichen Gründen werden Reaktoren mit einer möglichst großen Zahl von Kontaktrohren eingesetzt, wobei die Zahl der untergebrachten Kontaktrohre häufig im Bereich von 15000 bis 30000 liegt (vgl. DE-A-44 31 949).

Bezüglich des Wärmetauschmittelkreislaufs ist es bekannt, in jedem waagerechten Schnitt des Reaktors eine weitgehend homogene Temperaturverteilung des Wärmetauschmittels zu realisieren, um möglichst alle Kontaktrohre gleichmäßig am Reaktionsgeschehen zu beteiligen (z.B. DE-B-16 01 162). Der Glättung der Temperaturverteilung dient die Wärmezuführung bzw. Wärmeabführung über jeweils an den Reaktorenden angebrachten äußeren Ringleitungen mit einer Vielzahl von Mantelöffnungen, wie sie beispielsweise in DE-B-34 09 159 beschrieben sind.

Eine weitere Verbesserung des Wärmeüberganges wird durch den Einbau von Umlenkscheiben erreicht, die abwechselnd in der Reaktormitte und am Reaktorrand einen Durchtrittsquerschnitt freilassen. Eine derartige Anordnung ist insbesondere für ringförmig angeordnete Rohrbündel mit einem freien zentralen Raum geeignet und beispielsweise aus GB-B-31 01 75 bekannt.

In großen Reaktoren mit einer Zahl von Kontakrohren im oben angegebenen Bereich von etwa 15000 bis 30000, die zusätzlich mit Umlenkscheiben ausgestattet sind, ist der Druckverlust des Wärmetauschmittels vergleichsweise sehr groß. So muß die zum Abtransport der bei Oxidationsreaktionen freiwerdenden Wärme häufig verwendete eutektische Salzschmelze von Kaliumnitrat und Natriumnitrit, die bei einer Anwendungs-temperatur von ca. 350 bis 400°C eine wasserähnliche Viskosität aufweist, in einen Reaktor der oben erwähnten Größe mit einer Förderhöhe von ca 4 bis 5 m gepumpt werden, um den Druckverlust zu überwinden.

Zweckmäßigerweise wird bei derartigen großen Reaktoren das Pumpsystem zwischen der oberen und der unteren Ringleitung angeordnet, wobei das Wärmetauschmittel in den unteren Bereich des Reaktors, beispielsweise über eine Ringleitung, zugeführt wird.

Würde bei derartigen großen Reaktoren die Salzschmelze direkt in den oberen Reaktorteil oder die obere Ringleitung gepumpt werden, so würde die erforderliche Förderhöhe von 4 bis 5 m ein technisch ungünstiges und störanfälliges Pumpsystem erfordern, unter anderem wegen aufwendiger Pumpenwellenabdichtungen, längerer Pumpenwellen, sowie größerem Wärme-eintrag durch die Pumpenwelle in die untere Motorlagerung. Weiterhin würde die obengenannte Förderhöhe ein hochgestelltes Salzschmelze-Ausgleichsgefäß erfordern, das aus Sicherheitsgründen unerwünscht ist.

Die Zuführung des Wärmetauschmittels am oberen Reaktorende, d.h. im Gleichstrom mit dem ebenfalls am oberen Reaktorende in die Kontaktrohre zugeleiteten Reaktionsgemisch ist bekanntermaßen für die Reaktionsführung vorteilhaft (vgl. DE-A-44 31 449).

Die Gleichstromführung hat gegenüber der Gegenstromfahrweise Vorteile, wie höhere Durchsätze, niedrigere Katalysator-Hotspot-Temperaturen, erwünschter Anstieg der Wärmetauschmitteltemperatur in Richtung der Endreaktion in den Kontaktrohren, gute Temperaturgleichförmigkeit des Wärmetauschmittels über den Reaktorquerschnitt, d.h. gute waagerechte Temperaturschichtung, eindeutige Betriebzustände über die Höhe des Kontaktrohrraumes wegen fehlender Rückkopplung durch das Wärmetauschmittel.

Eine Gleichstromführung von Reaktionsgemisch und Wärmetauschmittel, wie in DE-A-44 31 449 beschrieben oder in DE-A-22 01 528, Figur 1 dargestellt, stößt jedoch bezüglich des Pumpsystems auf die oben genannten Schwierigkeiten, sofern das Wärmetauschmittel dem oberen Reaktorbereich, beispielsweise direkt über eine obere Ringleitung, zugeführt und aus dem unteren Reaktorbereich, beispielsweise direkt über eine Ringleitung, abgeführt wird.

Es ist somit Aufgabe der Erfindung, einen Reaktor zur Verfügung zu stellen, der die genannten Nachteile bezüglich des Pumpsystems nicht aufweist. Das Pumpsystem soll gegenüber der für große Reaktoren mit einer Vielzahl von Kontaktroheen beispielsweise bis zu 40000, insbesondere von 15000 bis 30000 Kontaktrohren, bewährten Bauweise mit Zuführung des Wärmetauschmittels im unteren Reaktorbereich in die untere Ringleitung und Abführung aus der oberen Ringleitung nicht abgeändert werden; dabei soll das Wärmetauschmittel dennoch die Kontaktrohre im Gleichstrom mit dem durch die Kontaktrohre geleiteten Reaktionsgemisch umströmen.

Die Erfindung geht aus von einem Reaktor mit einem Kontaktrohrbündel, durch dessen die Kontaktrohre umgebenden Raum ein Wärmetauschmittelkreislauf geleitet wird, mit Ringleitungen an beiden Reaktorenden mit Mantelöffnungen für die Zu- bzw. Abführung eines Wärmetauschmittels mittels einer oder mehrerer Pumpen gegebenenfalls unter Überleitung des Wärmetauschmittels oder eines Teilstroms des Wärmetauschmittels über einen oder mehrere außenliegende Wärmetauscher, wobei das Wärmetauschmittel der unteren Ringleitung zugeführt und über die obere Ringleitung zur (zu den) Pumpe(n) zurückgeführt wird, sowie mit Umlenkscheiben, die abwechselnd in der Reaktormitte und am Reaktorrand einen Durchtrittsquerschnitt freilassen.

Die Erfindung ist dann dadurch gekennzeichnet, daß die obere und untere Ringleitung jeweils mittels einer zylindermantelförmigen Zwischenwand in eine innere und eine äußere Ringleitung geteilt sind, und daß das Wärmetauschmittel der äußeren unteren Ringleitung, über einen Bereich außerhalb des Reaktors der inneren oberen Ringleitung, über deren Mantelöffnungen dem die Kontaktrohre umgebenden Raum zugeführt, über Mantelöffnungen in die innere untere Ringleitung und anschließend über einen Bereich außerhalb des Reaktors über die äußere obere Ringleitung abgeführt wird.

Es wurde gefunden, daß der Raum zwischen oberer und unterer Ringleitung zur Umlenkung des Wärmetauschmittels genutzt werden kann, wobei der Vorteil einer Gleichstromführung von Wärmetauschmittel und Reaktionsgemisch mit der bewährten Pumpenanordnung mit Zuführung des Wärmetauschmittels zur unteren Ringleitung verbunden werden kann.

Dazu ist erfindungsgemäß jeweils eine zylindermantelfönnige Zwischenwand in der oberen sowie in der unteren Ringleitung angeordnet, die diese jeweils in eine innere und eine äußere Ringleitung trennt. Das Wärmetauschmittel wird nun der äußeren unteren Ringleitung zugeführt, die über den Bereich zwischen oberer und unterer Ringleitung mit der inneren oberen Ringleitung verbunden ist, von hier in bekannter Weise über Mantelöffinungen in den die Kontaktrohre umgebenden Raum geleitet, wobei über Umlenkscheiben in bekannter Weise eine mäanderförmige Strömung ausgebildet wird. Das Wärmetauschmittel verläßt über Mantelöffnungen in bekannter Weise den die Kontaktrohre umgebenden Raum im unteren Reaktorteil und tritt in die untere innere Ringleitung ein. Diese ist wiederum über den Bereich zwischen oberer und unterer Ringleitung mit der oberen äußeren Ringleitung verbunden.

In vorteilhafter Weise ist der Bereich zwischen oberer und unterer Ringleitung durch einen Zylindermantel geschlossen, wobei ein Hohlzylinder entsteht, der durch radiale Zwischenwände, die auf der Reaktorgrundfläche senkrecht stehen, in Kammern aufgeteilt wird, deren Trennwände zu den Ringleitungen alternierend innere und äußere Kreisring-Ausschnitte freilassen, wobei in Draufsicht stets ein offener Kreisring-Ausschnitt über einem geschlossenen Kreisring-Ausschnitt und umgekehrt angeordnet ist. Die Kammern werden somit stets von unten nach oben, alternierend von Wärmetauschmittel, das von der (den) Pumpe(n) kommt, und von Wärmetauschmittel, das aus dem Reaktorraum kommt, durchströmt.

Die Anzahl der Kammern ist grundsätzlich nicht begrenzt, aus Zweckmäßigkeitsgründen kann eine Zahl von 12 bis 96, bevorzugt von 24 bis 48, vorgesehen werden, so daß alternierend je 12 bis 24 Kammern (entsprechend 3 bis 6 Kammern je Viertelumfang) für den Transport (das Umdirigieren) des Wärmetauschmittels zum Eintritt im oberen Bereich des die Kontaktrohre umgebenden Reaktorraums bzw. zur Abführung aus dem unteren Bereich desselben zur Verfügung stehen.

Die zylindermantelförmigen Zwischenwände, die die obere sowie die untere Ringleitung jeweils in eine innere und eine äußere Ringleitung auftrennen, können grundsätzlich jeden Durchmesser aufweisen, der zwischen dem Außen- und dem Innendurchmesser der Ringleitungen liegt, in bevorzugter Weise ist jedoch der Durchmesser der zylindermantelförmigen Zwischenwände kleiner oder gleich dem arithmetischen Mittelwert von Außen- und Innendurchmesser der Ringleitungen.

Gemäß einer bevorzugten Ausführungsform ist in mindestens einem Teil der Kammern, die von Wärmetauschmittel durchströmt werden, das zur (zu den) Pumpe(n) abgeführt wird, jeweils eine Bypass-Kammer mit Mantelöffnung zum Reaktorraum sowie mit einer Regulierplatte im Bereich der äußeren oberen Ringleitung angeordnet, wobei die Position der Regulierplatte über einen Stellantrieb und eine Antriebsspindel in Richtung der Reaktorlängsachse verstellbar ist. In dieser Ausgestaltung kann ein regelbarer Teilstrom des aus dem Reaktorraum kommenden Wärmetauschmittels bereits auf mittlerer Höhe des Reaktors abgezogen werden, so daß der untere Teil des die Kontaktrohre umgebenden Reaktorraums nur noch von der verbleibenden Teilmenge des Wärmetauschmittels durchströmt wird. Diese Ausführungsform ist optimiert bezüglich der nachlassenden Wärmeentwicklung im unteren Teil der Kontaktrohre. Zudem wird eine Verringerung des Druckverlustes erzielt, was eine reduzierte Pumpenleistung und somit eine erhöhte Wirtschaftlichkeit ermöglicht.

Der Reaktor ist nicht eingeschränkt bezüglich der Art des Wärmetauschmittels; dieses kann gleichermaßen zur Abführung von Wärme, d.h. zur Durchführung exothermer Reaktionen, wie auch für die Zuführung von Wärme an das die Kontaktrohre durchströmende Reaktionsgemisch, d.h. zur Durchführung endothermer Reaktionen, eingesetzt werden.

Der Reaktor ist besonders geeignet zur Durchführung von Oxidationsreaktionen, insbesondere zur Herstellung von Phthalsäureanhydrid, Maleinsäureanhydrid, Glyoxal, (Meth)acrolein und (Meth)acrylsäure.

Die Erfindung wird im folgenden anhand von Ausführungsbeispielen und einer Zeichnung näher erläutert. Es zeigen im einzelnen:

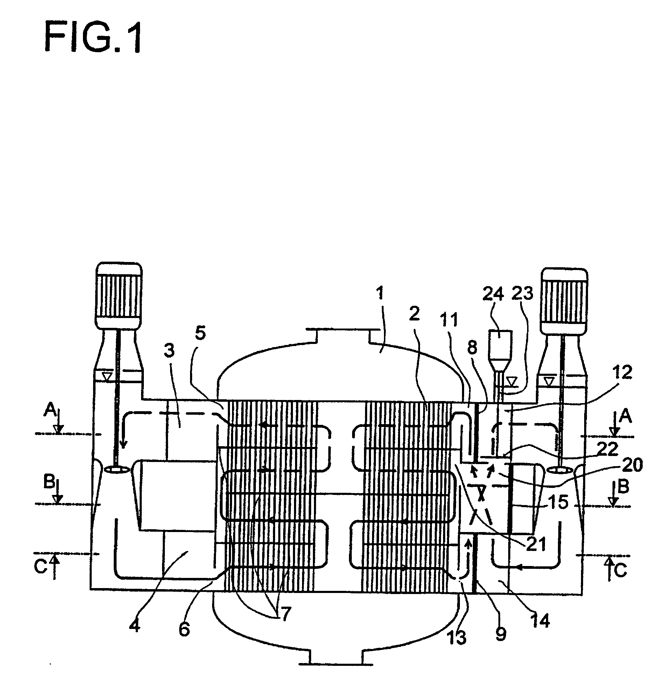

- Figur 1, rechte Seite:: einen Längsschnitt durch einen Reaktor mit Wärmetauschmittelkreislauf gemäß der Erfindung,
- Figur 1, linke Seite:: einen Längsschnitt durch einen Reaktor mit Wärmetauschmittelkreislauf nach dem Stand der Technik,
- Figur 2, rechte Seite:: einen Querschnitt durch einen erfindungsgemäßen Reaktor im Bereich der Trennfläche zwischen oberer Ringleitung und mittlerem Hohlzylinder (Schnitt A-A),

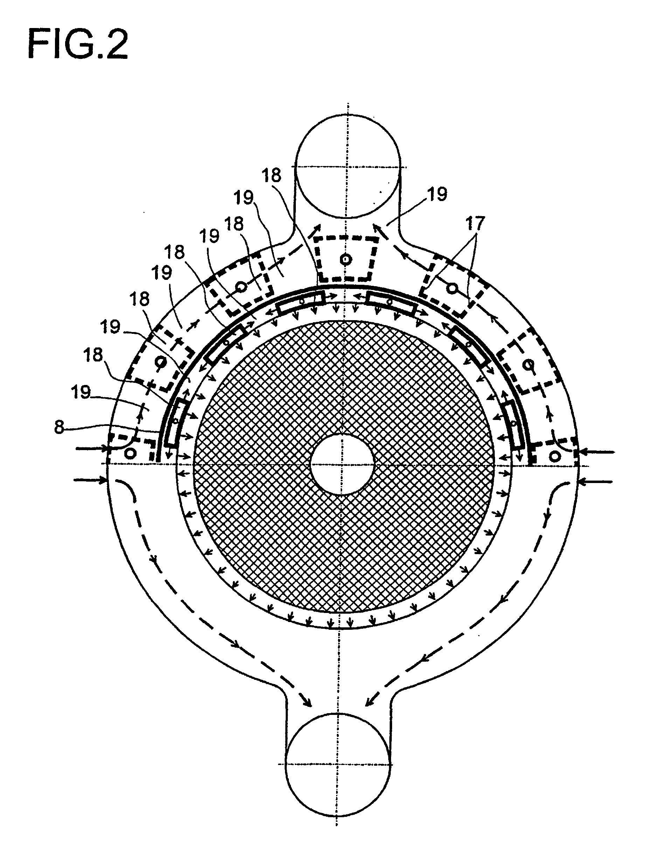

- Figur 2, linke Seite:: einen Querschnitt in der Ebene A-A durch einen Reaktor nach dem Stand der Technik,

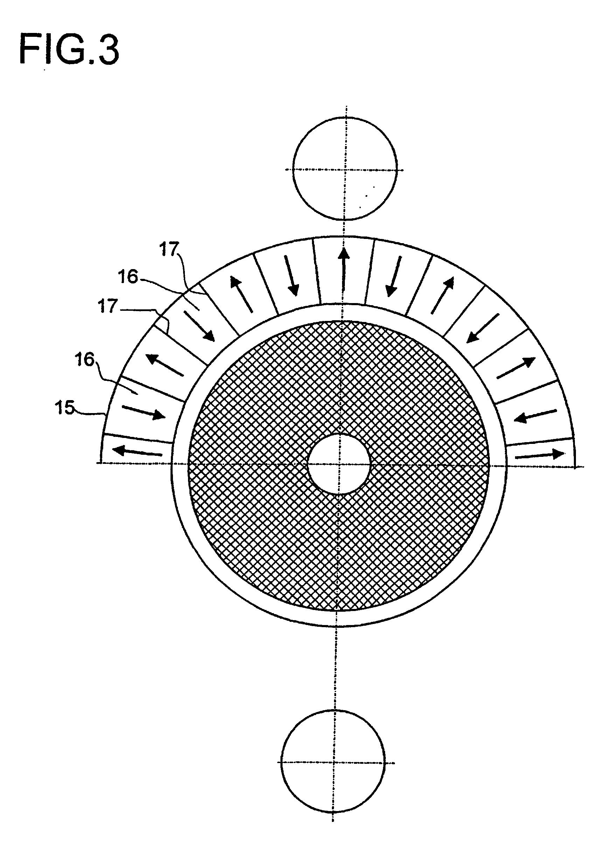

- Figur 3, rechte Seite:: einen Querschnitt durch einen erfindungsgemäßen Reaktor im Bereich des mittleren Hohlzylinders (Schnitt B-B),
- Figur 3, linke Seite:: einen Querschnitt in der Ebene B-B durch einen Reaktor nach dem Stand der Technik,

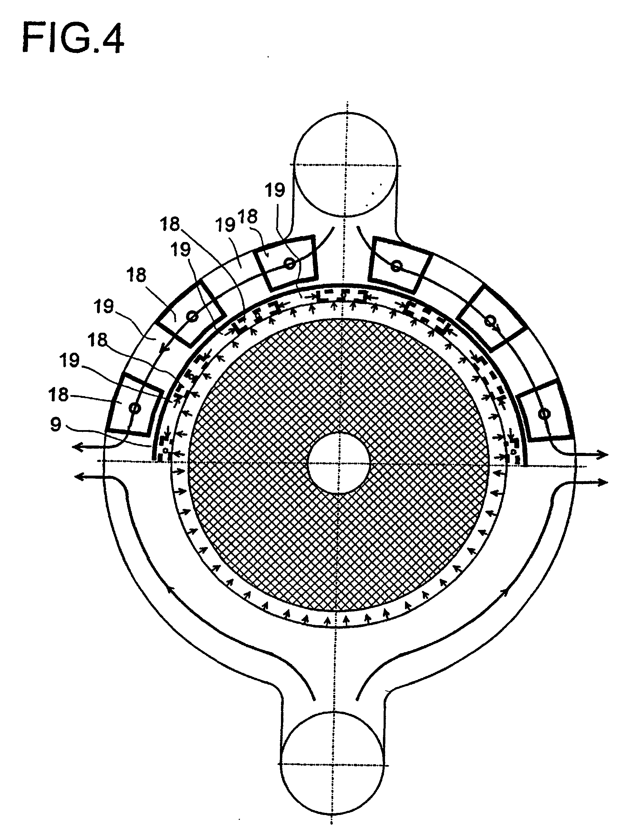

- Figur 4, rechte Seite:: einen Querschnitt durch einen erfindungsgemäßen Reaktor im Bereich der Trennfläche zwischen mittlerem Hohlzylinder und unterer Ringleitung (Schnitt C-C),
- Figur 4, linke Seite:: einen Querschnitt in der Ebene C-C durch einen Reaktor nach dem Stand der Technik,

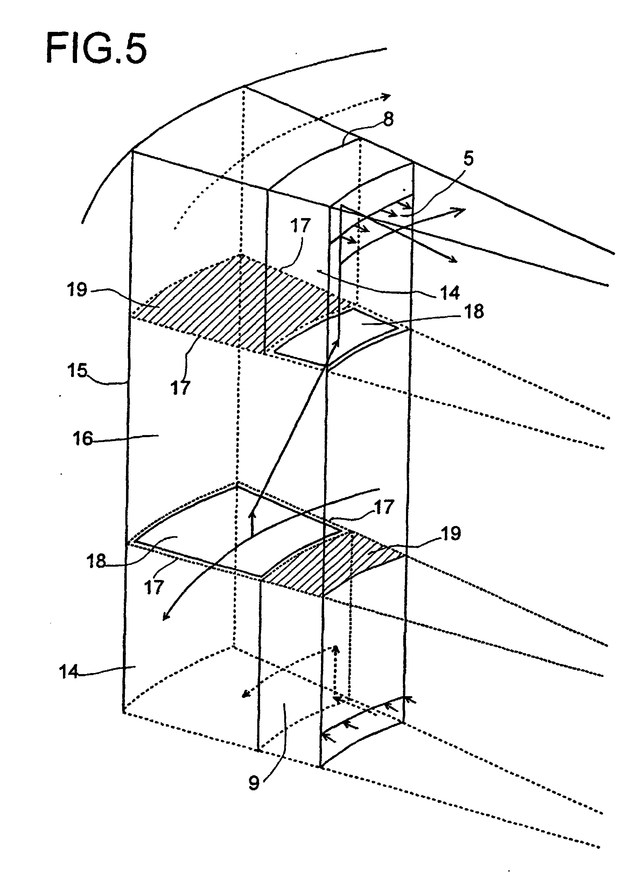

- Figur 5:: einen Ausschnitt aus einem erfindungsgemäßen Reaktor zur Erläuterung der Wärmetauschmittelzuführung zum die Kontaktrohre umgebenden Raum,

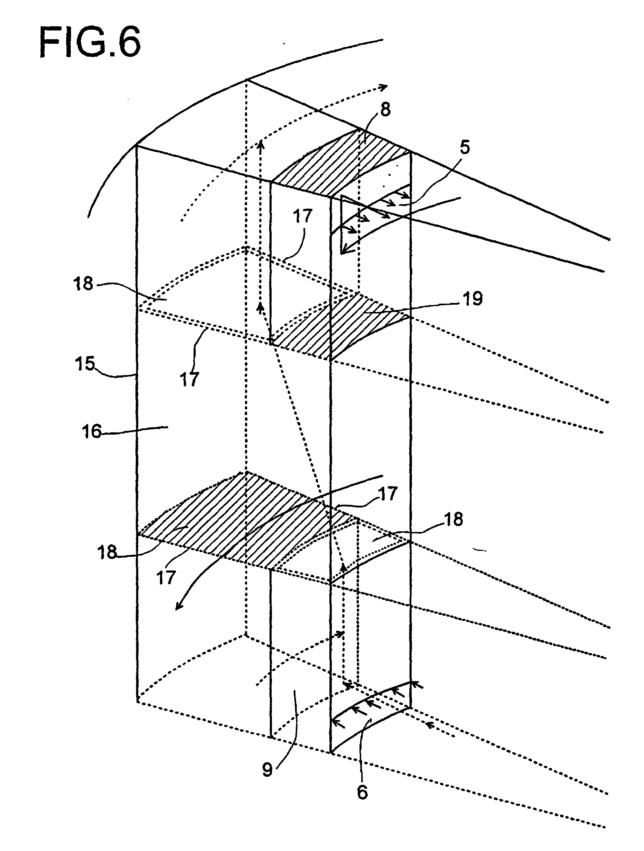

- Figur 6:: einen Ausschnitt aus einem erfindungsgemäßen Reaktor zur Erläuterung der Wännetauschmittelabführung aus dem die Kontaktrohre umgebenden Raum zur (zu den) Pumpe(n),

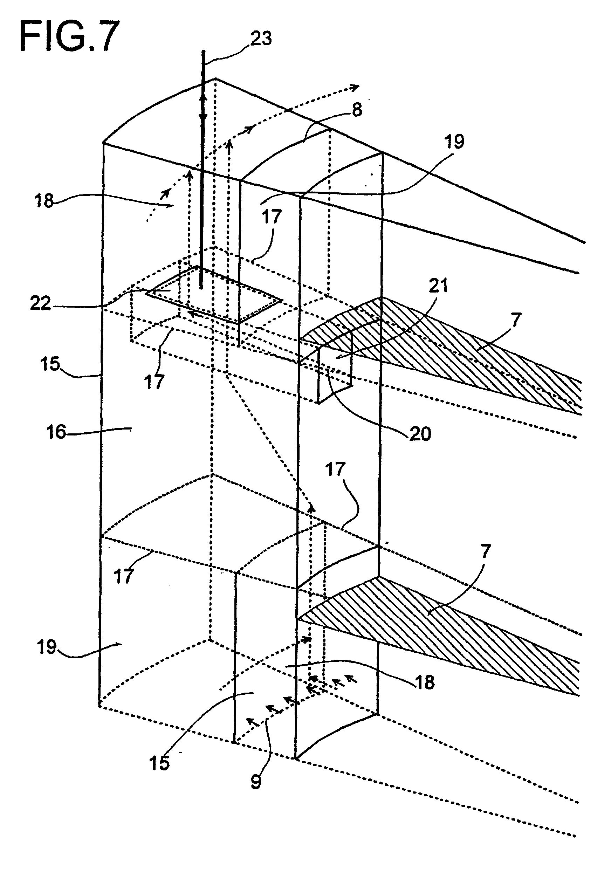

- Figur 7:: einen Ausschnitt aus einer bevorzugten Ausführungsform eines erfindungsgemäßen Reaktors und

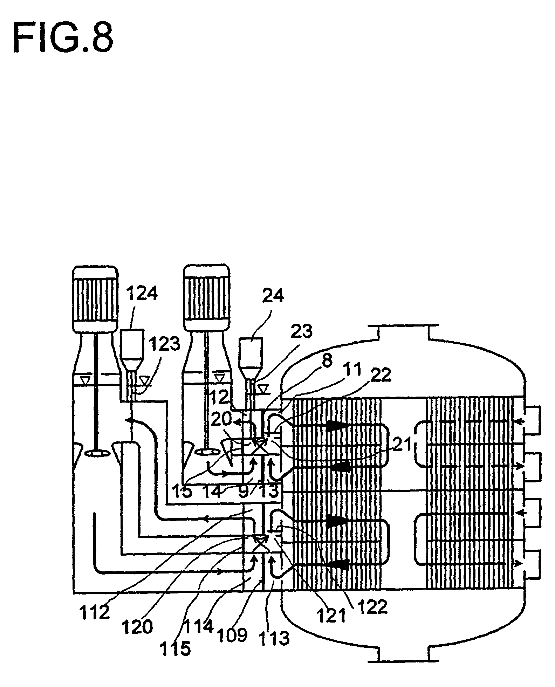

- Figur 8:: einen Querschnitt durch einen erfindungsgemäßen Reaktor mit zwei Wärmetauschmittelkreisläufen.

Figur 1 zeigt einen zylinderförmigen Reaktor 1 mit einem vertikalen Kontaktrohrbündel 2, das in der Zylindermitte einen Innenraum freiläßt, mit unterer Ringleitung 4, der Wärmetauschmittel zugeführt wird, sowie mit oberer Ringleitung 3, über die Wärmetauschmittel abgeführt wird, wobei die Zu- bzw. Abführung des Wärmetauschmittels über Mantelöffnungen 5 und 6 erfolgt, sowie mit Umlenkscheiben 7, die einen maanderförmigen Wärmetauschmittelkreislauf bewirken.

Insoweit ist der Aufbau des Reaktors nach dem Stand der Technik (linke Seite der Figur 1) mit dem Aufbau des Reaktors nach der Erfindung (rechte Seite der Figur 1) gleich.

Wie auf der rechten Seite der Figur 1 ersichtlich, weist der erfindungsgemäße Reaktor gegenüber dem Stand der Technik folgende Abänderungen auf:

Die obere Ringleitung 3 ist durch eine zylindermantelförmige Zwischenwand 8 in eine innere obere Ringleitung 11 und eine äußere obere Ringleitung 12 getrennt; analog ist die untere Ringleitung 4 mittels der zylindermantelförmigen Zwischenwand 9 in eine innere untere Ringleitung 13 und eine äußere untere Ringleitung 14 getrennt.

Vorzugsweise ist der Bereich zwischen den Ringleitungen 3 und 4 mittels eines Zylindermantels 15 zu einem Hohizylinder geschlossen. In diesem Bereich erfolgt die Umlenkung des Wärmetauschmittels von der äußeren unteren Ringleitung 14 zur inneren oberen Ringleitung 11 bzw. von der inneren unteren Ringleitung 13 zur äußeren oberen Ringleitung 12. Diese ümlenkung des Wärmetauschmittels im Bereich des mittleren Hohlzylinders erfolgt in bevorzugter Weise durch Ausbildung von Kammern 16 mittels radialer Zwischenwände 17, die auf der Reaktorgrundfläche senkrecht stehen (Figur 3).

Die Führung des Wärmetauschmittels im Bereich der Trennflächen des mittleren Hohlzylinders zur oberen bzw. unteren Ringleitung ist aus den in Figur 2, rechts bzw. Figur 4, rechts, dargestellten Querschnitten ersichtlich: Durch die zylindermantelförmigen Zwischenwände 8 bzw. 9 und die radialen Zwischenwände 17 ergeben sich innere und äußere Kreisring-Ausschnitte. Diese sind nun erfindungsgemäß, wie in Figur 2, rechts bzw. in Figur 4, rechts, dargestellt, alternierend als offene Kreisring-Ausschnitte 18 bzw. geschlossene Kreisring-Ausschnitte 19 ausgebildet. Alternierend heißt in diesem Zusammenhang, daß auf jeder inneren bzw. äußeren Ringleitung auf einen offenen Kreisring-Ausschnitt jeweils ein geschlossener Kreisring-Ausschnitt folgt, und daß zusätzlich in Draufsicht stets ein offener Kreisring-Ausschnitt 18 in der Querschnittsdarstellung A-A einem geschlossenen Kreisring-Ausschnitt 19 in der Querschnittsdarstellung C-C und umgekehrt entspricht. Durch diese Ausgestaltung werden die Kammern stets von unten nach oben, alternierend von Wärmetauschmittel, das von der (den) Pumpe(n) kommt, und von Wärmetauschmittel, das aus dem Reaktorraum kommt, wie in Figur 3 dargestellt, durchströmt.

Zur Verdeutlichung der Wärmetauschmittelführung sind in den Figuren 5 und 6 Ausschnitte aus einem erfindungsgemäßen Reaktor dargestellt:

In Figur 5 ist eine Kammer 16 dargestellt, durch die Wärmetauschmittel von der (den) Pumpe(n) über die untere äußere Ringleitung 14 durch den offenen Kreisringausschnitt 18 in die Kammer 16 einströmt, diese von unten nach oben durchströmt, im oberen Bereich über einen weiteren offenen Kreisringausschnitt 18 verläßt, in die obere innere Ringleitung 11 einströmt und über die Mantelöffnung 5 dem die Kontaktrohre umgebenden Reaktorraum zugeführt wird.

Die in Figur 6 dargestellte Kammer 16, die unmittelbar an die in Figur 5 dargestellte Kammer 16 angrenzt, ist dagegen von Wärmetauschmittel, das vom die Kontaktrohre umgebenden Reaktorraum über die Mantelöffnung 6 durch die untere innere Ringleitung 13 und den offenen Kreisringausschnitt 18 führt, durchströmt. Aus der Kammer 16 wird das Wärmetauschmittel durch einen weiteren offenen Kreisringausschnitt 18 in die obere äußere Ringleitung 12 und von hier zur (zu den) Pumpe(n) abgeführt.

Die Figur 7 zeigt eine bevorzugte Ausführungsform des erfindungsgemäßen Reaktors. Gemäß dieser Ausführungsform ist in mindestens einem Teil der Kammern 16, die von Wärmetauschmittel durchströmt werden, das zur (zu den) Pumpe(n) abgeführt wird, jeweils eine zusätzliche Bypass-Kammer 20 mit einer Mantelöffnung 21 zum Reaktorraum sowie eine Regulierplatte 22 im Bereich der Trennwand der Kammer 16 zur oberen äußeren Ringleitung 12 angeordnet. Die Position der Regulierplatte 22 kann in Richtung der Reaktorlängsachse über einen geeigneten Stellantrieb und eine Antriebsspindel 23 verändert werden.

Die Figur 8 zeigt eine bevorzugte Ausführungsform eines erfindungsgemäßen Reaktors mit zwei Wärmetauschmittelkreisläufen. Der Reaktor ist analog einem Reaktor mit einem einzigen Wärmetauschmittelkreislauf, entsprechend Figur 1, rechte Seite aufgebaut. Im zweiten Wärmetauschmittelkreislauf sind jeweils dem ersten Wärmetauschmittelkreislauf entsprechende Merkmale mit entsprechenden Bezugsziffern gekennzeichnet.

Analog ist es möglich den erfindungsgemäßen Reaktor um weitere Wärmetauschmittelkreisläufe zu ergänzen. Der Reaktor kann dadurch dem jeweiligen konkreten Wärmeprofil der durchzuführenden Reaktion optimal angepaßt werden.

## Patentansprüche

1. Reaktor (1) mit einem Kontaktrohrbündel (2), durch dessen die Kontaktrohre umgebenden Raum ein Wärmetauschmittelkreislauf geleitet wird, mit Ringleitungen (3, 4) an beiden Reaktorenden mit Mantelöffnungen (5, 6) für die Zu- bzw. Abführung eines Wärmetauschmittels mittels einer oder mehrerer Pumpen gegebenenfalls unter Überleitung des Wärmetauschmittels oder eines Teilstroms des Wärmetauschmittels über einen oder mehrere außenliegende Wärmetauscher, wobei das Wärmetauschmittel der unteren Ringleitung (4) zugeführt und über die obere Ringleitung (3) zur (zu den) Pumpe(n) zurückgeführt wird, sowie mit Umlenkscheiben (7), die abwechselnd in der Reaktormitte und am Reaktorrand einen Durchtrittsquerschnitt freilassen, **dadurch gekennzeichnet, daß** die obere (3) und untere (4) Ringleitung jeweils mittels einer zylindermantelförmigen Zwischenwand (8, 9) in eine innere (11, 13) und eine äußere (12, 14) Ringleitung geteilt sind, und daß das Wärmetauschmittel der äußeren unteren Ringleitung (14), über einen Bereich außerhalb des Reaktors der inneren oberen Ringleitung (11), über deren Mantelöffnungen (5) dem die Kontaktrohre (2) umgebenden Raum zugeführt, über die Mantelöffnungen (6) in die innere untere Ringleitung (13) und anschließend über einen Bereich außerhalb des Reaktors über die äußere obere Ringleitung (12) abgeführt wird.

2. Reaktor (1) nach Anspruch 1, **dadurch gekennzeichnet, daß** der Bereich zwischen unterer (4) und oberer (3) Ringleitung durch einen Zylindermantel (15) geschlossen wird, wobei ein Hohlzylinder entsteht, der durch radiale Zwischenwände (17), die auf der Reaktorgrundfläche senkrecht stehen, in Kammern (16) aufgeteilt wird, deren Trennwände zu den Ringleitungen (3, 4) alternierend innere und äußere Kreisring-Ausschnitte freilassen, wobei in Draufsicht stets ein offener Kreisring-Ausschnitt (18) über einem geschlossenen Kreisring-Ausschnitt (19) und umgekehrt angeordnet ist.

3. Reaktor (1) nach Anspruch 1 oder Anspruch 2, **dadurch gekennzeichnet, daß** die Anzahl der Kammern (16) 12 bis 96, bevorzugt 24 bis 48, beträgt.

4. Reaktor (1) nach einem der Ansprüche 1 bis 3, **dadurch gekennzeichnet, daß** der Durchmesser der zylindermantelförmigen Zwischenwände (8, 9) kleiner oder gleich dem arithmetischen Mittelwert von Außen- und Innendurchmesser der Ringleitungen (3, 4) ist.

5. Reaktor (1) nach einem der Ansprüche 1 bis 4, **dadurch gekennzeichnet, daß** in mindestens einem Teil der Kammern (16), die von Wärmetauschmittel durchströmt werden, das zur (zu den) Pumpe(n) abgeführt wird, jeweils eine Bypass-Kammer (20) mit Mantelöffnung (21) zum Reaktorraum sowie mit einer Regulierplatte (22) im Bereich der äußeren oberen Ringleitung (12) angeordnet ist, wobei die Regulierplatte (22) über einen Stellantrieb (24) und eine Antriebsspindel (23) in Richtung der Reaktorlängsachse verstellbar ist.

6. Reaktor (1) nach einem der Ansprüche 1 bis 5, **dadurch gekennzeichnet, daß** durch den die Kontaktrohre umgebenen Raum zwei oder mehrere Wärmetauschmittelkreisläufe geleitet werden.

7. Verwendung des Reaktors nach einem der vorhergehenden Ansprüche zur Durchführung von Oxidationsreaktionen, insbesondere zur Herstellung von Phthalsäureanhydrid, Maleinsäureanhydrid, Glyoxal, (Meth)acrolein oder (Meth)acrylsäure.

## Claims

1. A reactor (1) having a contact tube bundle (2) through whose space surrounding the contact tubes a heat-exchange medium circuit is passed, with ring lines (3, 4) at both ends of the reactor with jacket apertures (5, 6) for the supply and discharge of a heat-exchange medium by means of one or more pumps, if desired with the heat-exchange medium or a substream of the heat-exchange medium being passed through one or more external heat exchangers, in which case the heat-exchange medium is fed to the lower ring line (4) and fed back to the pump(s) via the upper ring line (3), and with baffle plates (7) which leave a passage cross section alternately in the reactor center and the reactor edge, wherein the upper (3) and lower (4) ring lines are each divided into an inner (11, 13) and outer (12, 14) ring line by means of a cylindrical partition wall (8, 9), and the heat-exchange medium is fed through the outer lower ring line (14), via a region outside the reactor to the inner upper ring line (11), via the latter's jacket apertures (5) to the space surrounding the contact tubes (2), via the jacket apertures (6) into the inner lower ring line (13) and subsequently via a region outside the reactor is discharged via the outer upper ring line (12).

2. A reactor (1) as claimed in claim 1, wherein the region between the lower (4) and upper (3) ring lines is closed by a cylinder envelope (15), forming a hollow cylinder, which is divided, by radial partition walls (17) perpendicular to the reactor base, into chambers (16) whose dividing walls to the ring lines (3, 4) leave alternately inner and outer circular ring sections, where, in plan view, an open circular ring section (18) is always arranged above a closed circular ring section (19), and vice versa.

3. A reactor (1) as claimed in claim 1 or 2, wherein the number of chambers (16) is from 12 to 96, preferably from 24 to 48.

4. A reactor (1) as claimed in any one of claims 1 to 3, wherein the diameter of the cylindrical partition walls (8, 9) is less than or equal to the arithmetic mean of the outer and inner diameters of the ring lines (3, 4).

5. A reactor (1) as claimed in any one of claims 1 to 4, wherein a bypass chamber (20) with jacket aperture (21) to the reactor space and a regulating plate (22) is arranged in the region of the outer upper ring line (12) in each case in at least some of the chambers (16) through which heat-exchange medium flows and is discharged to the pump(s), the regulating plate (22) being adjustable in the direction of the longitudinal axis of the reactor via an actuating drive (24) and a drive spindle (23).

6. A reactor (1) as claimed in any one of claims 1 to 5, wherein two or more heat-exchange medium circuits are passed through the space surrounding the contact tubes.

7. Use of a reactor as claimed in any one of the preceding claims for carrying out oxidation reactions, in particular for the preparation of phthalic anhydride, maleic anhydride, glyoxal, (meth)acrolein or (meth)acrylic acid.

## Revendications

1. Réacteur (1) comportant un faisceau de tubes de contact (2) et un circuit contenant un milieu échangeur de chaleur que l'on conduit au travers de l'espace qui entoure les tubes de contact du faisceau, muni des canalisations circulaires (3, 4) au niveau des deux extrémités du réacteur présentant des ouvertures de chemise (5, 6) destinées respectivement à l'amenée et à l'évacuation d'un milieu échangeur de chaleur au moyen d'une ou de plusieurs pompes, éventuellement en détournant par un ou plusieurs échangeurs de chaleur extérieurs, le milieu échangeur de chaleur ou un courant partiel du milieu échangeur de chaleur, procédé lors duquel on amène le milieu échangeur de chaleur par l'intermédiaire de la canalisation inférieure (4) et lors duquel on le recycle, par l'intermédiaire de la canalisation circulaire supérieure (3), dans la pompe ou dans les pompes, et muni de plaques de déviation (7) qui laissent ouverte, en alternance au centre du réacteur et au bord du réacteur, une section transversale de passage, **caractérisé en ce que** les canalisations circulaires supérieure (3) et inférieure (4) sont divisées, respectivement au moyen d'une paroi intermédiaire en forme de chemise de cylindre (8, 9), en une canalisation circulaire intérieure (11, 13) et extérieure (12, 14), et **caractérisé en ce que** l'on procède à l'amenée du milieu échangeur de chaleur provenant de la canalisation inférieure extérieure (14) vers l'espace qui entoure les tubes de contact (2), en passant par une zone située à l'extérieur du réacteur, par l'intermédiaire de la canalisation circulaire supérieure intérieure (11), puis par les ouvertures de chemise (5) de cette dernière, et que l'on procède à son évacuation par les ouvertures de chemise (6) et par l'intermédiaire de la canalisation circulaire inférieure intérieure (13), et ensuite, en passant par une zone située à l'extérieur du réacteur, par l'intermédiaire de la canalisation circulaire supérieure extérieure (12).

2. Réacteur (1) selon la revendication 1, **caractérisé en ce que** l'on ferme, au moyen d'une chemise de cylindre (15), la zone entre la canalisation circulaire inférieure (4) et supérieure (3), en formant un cylindre creux que l'on divise en chambres (16), au moyen de parois intermédiaires radiales (17) qui sont disposées de manière verticale sur la surface de base du réacteur, chambres qui présentent, dans leurs parois intermédiaires des canalisations circulaires (3, 4), des secteurs d'anneau de cercle ouverts en alternance interne et externe, où, vu d'en haut, un secteur d'anneau de cercle ouvert (18) est toujours disposé par-dessus un secteur d'anneau de cercle fermé (19) et vice versa.

3. Réacteur (1) selon la revendication 1 ou la revendication 2, **caractérisé en ce que** le nombre de chambres (16) est compris entre 12 et 96, de préférence entre 24 et 48.

4. Réacteur (1) selon l'une quelconque des revendications 1 à 3, **caractérisé en ce que** le diamètre des parois en forme de chemise de cylindre (8, 9) est inférieur ou égal à la moyenne arithmétique des diamètres extérieur et intérieur des canalisations circulaires (3, 4).

5. Réacteur (1) selon l'une quelconque des revendications 1 à 4, **caractérisé en ce que** l'on dispose dans chaque chambre, dans au moins une partie des chambres (16) qui sont soumises au passage du milieu échangeur de chaleur que l'on évacue vers la pompe ou vers les pompes, dans sa zone de la canalisation supérieure extérieure (12), une chambre de dérivation (20) présentant une ouverture de chemise (21) vers l'espace intérieur du réacteur ainsi qu'une plaque de réglage (22), la plaque de réglage (22) étant ajustable, dans la direction de l'axe longitudinale du réacteur, au moyen d'un mécanisme de commande (24) et d'une bielle de commande (23).

6. Réacteur (1) selon l'une quelconque des revendications 1 à 5, **caractérisé en ce que** l'on conduit au moins deux circuits contenant le milieu échangeur de chaleur au travers de l'espace qui entoure les tubes de contact.

7. Mise en ouvre du réacteur selon l'une quelconque des revendications précédentes, pour la préparation de l'anhydride phtalique, de l'anhydride maléique, du glyoxal, de la (méth)acroléine ou de l'acide (méth)acrylique.
